# DEMANDE DE BREVET EUROPEEN

(11) **EP 1 234 571 A1**
(43) Date de publication de la demande: **28.08.2002**
(21) Numéro de dépôt: 02290413.0
(22) Date de dépôt: 20.02.2002
(51) Int. Cl.: A61K 7/48

(54) **Composition pour application topique comprenant un hydroxystilbène et un polyol**

(30) Priorité: 21.02.2001 FR 0102353
(71) Demandeur: L'OREAL, 75008 Paris (FR)
(72) Inventeur: Baldo, Francine, 9330 Sceaux (FR); Roger, Véronique, 92220 Bagneux (FR)
(74) Mandataire: Catherine, Alain

(57) **Abrégé**

L'invention concerne une composition adaptée à une application topique sur la peau comprenant, dans un milieu physiologiquement acceptable, au moins un hydroxystilbène et au moins un polyol pour solubiliser l'hydroxystilbène, dans un rapport pondéral du polyol à l'hydroxystilbène d'au moins 150/1.

L'invention concerne également l'utilisation d'une telle composition pour la fabrication de produits de soin et/ou de maquillage de la peau et/ou capillaires.

## Description

La présente invention concerne une composition adaptée à une application topique sur la peau, comprenant, dans un milieu physiologiquement acceptable, au moins un hydroxystilbéne, de préférence le resvératrol, et au moins un polyol.

Les hydroxystilbènes sont des composés répondant à la formule générale (I) : 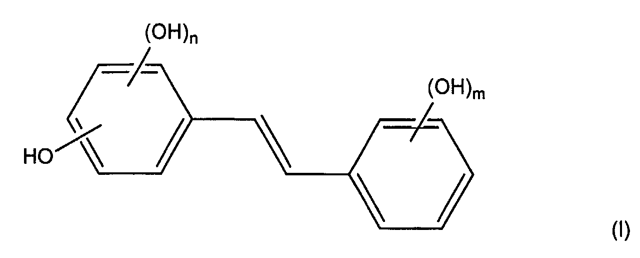 dans laquelle n est un nombre entier compris entre 0 et 4 inclusivement et m est un nombre entier compris entre 0 et 5 inclusivement. Ces composés peuvent être sous une forme cis ou trans.

Selon l'invention, le terme hydroxystilbène recouvre aussi bien les composés de formule (I) que leurs dérivés hydroxyalkylés.

Les hydroxystilbènes sont des composés que l'on retrouve à l'état naturel notamment dans les végétaux de la classe des spermatophytes et particulièrement dans la vigne et le raisin, et dans le vin.

Le resvératrol, ou 3, 4', 5 - trihydroxystilbène, fait partie des stilbènes qui sont des composés produits chez les plantes, essentiellement chez les spermatophytes, et appartiennent à la classe des molécules antibiotiques connues sous le nom de phytoalexines.

Le resvératrol existe naturellement dans de nombreuses plantes et fruits sous forme simple ou glucosylée. On trouve les deux formes, simple et glucosylée, en particulier dans la peau du raisin (Vrhovsek *et al*., Am. J. Enol. Vitic., vol. 48, n° 2, 1997) ou encore dans des surnageants de cultures *in vitro* de *vitis vinifera* (Teguo *et al*., *J.* Nat. Prod., 61, 655-657, 1998).

Le resvératrol est libéré en présence de glucosidases. Cette réaction se produit naturellement chez la plante, par exemple, an niveau de la peau du raisin. Au cours de la fermentation des vins rouges (fermentation alcoolique), cette réaction est réalisée par les glycosidases des levures, mais elle n'est pas totale et il reste une proportion importante de dérivés glucosylés. La forme glucosylée est présente en quantité variable selon les vins, certaines variétés de Pinot Noir contiennent exclusivement des hydroxystilbènes glucosylés (Soleas *et al*., Clinical Biochemistry, vol. 30, Mars 1997).

Différentes études *in vitro* et *in vivo* ont mis en évidence les propriétés biologiques intéressantes des hydroxystilbènes, en particulier leurs propriétés anti-inflammatoires, anti-oxydantes et anti-mutagènes, et leur influence sur le métabolisme des lipides et sur l'agrégation des plaquettes (Soleas et al., 1997 ; Jang et al., Science, vol. 275, 10 Janvier 1997).

Ces propriétés ont été mises à profit dans la réalisation de compositions cosmétiques contenant ces composés.

Par exemple, la demande de brevet internationale WO 99/04747 décrit des compositions cosmétiques comprenant du resvératrol, ainsi que leurs utilisations pour lutter contre les signes du vieillissement cutané, lisser la peau ou traiter les rides et les ridules

Malgré ces propriétés intéressantes, les hydroxystilbènes, et plus particulièrement, le resvératrol, présentent certains inconvénients, en raison de leur faible solubilité dans les solvants cosmétiques. En effet, les hydroxystilbènes ont tendance à recristalliser. Il s'ensuit une perte d'efficacité plus ou moins importante de ces composés dans les compositions en contenant, selon le degré de recristallisation. En outre, cette recristallisaton peut modifier la stabilité globale de ces compositions ainsi que leur aspect, ce qui pourrait détourner l'utilisateur de celles-ci.

La demanderesse a maintenant découvert que l'utilisation d'une grande quantité de polyols, éventuellement en association avec de l'éthanol, permettait d'éviter la recristallisation des hydroxystilbènes, en particulier du resvératrol, et ce, dans tous les supports cosmétiques classiquement utilisés, notamment les émulsions huile-dans-eau (H/E) ou eau-dans-huile (E/H), les nanoémulsions, les microémulsions, les gels aqueux, les gels anhydres, les solutions, ainsi que les bases oléosomes.

Par bases oléosomes, on entend, au sens de la demande, des émulsions du type huile-dans-eau formées de globules huileux pourvus d'un enrobage cristal liquide lamellaire, et dispersés dans une phase aqueuse. Ces bases sont décrites et revendiquées dans le brevet européen EP-0 641 557.

L'homme de l'art connaît l'utilisation d'hydroxystilbène dans des compositions cosmétiques ou pour la préparation de compositions cosmétiques et/ou adaptées à une application topique sur la peau.

En effet, la demande de brevet européen EP-0 953 344 décrit l'utilisation en tant que composé actif dans une composition ou pour la préparation d'une composition, d'une quantité efficace d'au moins un hydroxystilbène pour favoriser la desquamation de la peau, et/ou stimuler le renouvellement épidermique et/ou lutter contre le vieillissement de la peau. Rien n'est dit toutefois, dans ce document, sur la solubilisation de l'hydroxystilbène.

De même, la demande internationale WO 99/04747 décrit une composition de soin de la peau comprenant du resvératrol et un véhicule cosmétiquement acceptable. Cependant, cette demande ne concerne pas la solubilisation du resvératrol.

La présente invention a donc pour objet une composition adaptée à une application topique sur la peau comprenant, dans un milieu physiologiquement acceptable, au moins un hydroxystilbène et au moins un polyol, dans un rapport pondéral du polyol à l'hydroxystilbène d'au moins 150/1.

Selon l'invention, les hydroxystilbènes peuvent être utilisés seuls ou en mélanges de toute nature et peuvent être d'origine naturelle ou synthétique.

Parmi les hydroxystilbènes utilisables selon l'invention, on peut citer :
- le 4'-hydroxystilbène,
- le 2',4'-dihydroxystilbène,
- le 3',4'-dihydroxystilbène,
- le 4,4'-dihydroxystilbène,
- le 2',4',4-trihydroxystilbène,
- le 3',4',4-trihydroxystilbène,
- le 2,4,4'-trihydroxystilbène,
- le 3,4,4'-trihydroxystilbène,
- le 3,4',5-trihydroxystilbène,
- le 2',3,4-trihydroxystilbène,
- le 2,3',4-trihydroxystilbène,
- le 2',2,4'-trihydroxystilbène,
- le 2,4,4',5-tétrahydroxystilbène,
- le 2',3,4',5-tétrahydroxystilbène,
- le 2,2',4,4'-tétrahydroxystilbène,
- le 3,3',4',5-tétrahydroxystilbène,
- le 2,3',4,4'-tétrahydroxystilbène,
- le 3,3',4,4'-tétrahydroxystilbène,
- le 3,3',4',5,5'-pentahydroxystilbène,
- le 2,2',4,4',6-pentahydroxystilbène,
- le 2,3',4,4',6-pentahydroxystilbène, et
- le 2,2',4,4',6,6'-hexahydroxystilbène.

Préférentiellement, on utilise selon l'invention le 3,4',5-trihydroxystilbène, également appelé resvératrol.

La quantité d'hydroxystilbène utilisable selon l'invention dépend bien évidemment de l'effet recherché. A titre d'exemple, la quantité d'hydroxystilbène utilisable selon l'invention peut aller par exemple de 0,001% à 10%, et de préférence de 0,005% à 0,5% du poids total de la composition.

Les polyols peuvent être notamment choisis parmi la glycérine, les glycols, tels que le mono- ou di-propylène glycol, le butylène glycol, le pentylène glycol, et les polyéthylènes glycols, en particulier comportant de 4 à 8 motifs oxyde d'éthylène, et leurs mélanges.

Les polyols particulièrement préférés sont les polyéthylène glycols, en particulier le polyéthylène glycol 8 OE, le butylène-1,3-glycol, le 5-[2-(4-hydroxyphényl)vinyl]benzène-1, 3-diol et l'octyl -2-dodécanol.

De préférence, les compositions selon l'invention comprennent en outre un alcanol ayant de 1 à 6 atomes de carbone, en particulier de l'éthanol.

La quantité d'alcanol présent dans la composition peut atteindre jusqu'à 10% en poids, de préférence 5% en poids par rapport au poids total de la composition.

La composition selon l'invention peut être constituée par une émulsion, notamment huile-dans-eau (H/E) ou eau-dans-huile (E/H), voire sous forme d'une émulsion multiple.

La composition selon l'invention peut également être constituée par une émulsion du type huile-dans-eau formée de globules huileux pourvus d'un enrobage cristal liquide lamellaire, et dispersés dans une phase aqueuse.

Chaque globule huileux, de taille inférieure à 500 nanomètres et de préférence inférieure à 300 nanomètres, est enrobé d'une couche monolamellaire ou oligolamellaire obtenue à partir d'au moins un agent tensioactif lipophile, d'au moins un agent actif hydrophile et d'au moins un acide gras.

Par couche oligolamellaire, on entend, au sens de la demande, une couche comprenant de 2 à 5 lamelles lipidiques.

La phase aqueuse contient à l'état dissous l'hydroxystilbène et le polyol solubilisant.

Ce type d'émulsion, également appelé base oléosome, est décrit dans le brevet européen EP-0 641557.

La composition selon l'invention peut comprendre une phase huileuse à base d'huile animale, végétale, minérale, siliconée, fluorée et/ou synthétique.

La phase huileuse peut également comprendre au moins un alcool gras ou au moins un acide gras, ainsi qu'au moins un tensioactif.

On peut notamment citer les huiles hydrocarbonées telles que l'huile de paraffine ou de vaseline ; le perhydrosqualène ; le beurre de karité ; l'huile d'arara, l'huile d'amande douce, de calophyllum, de palme, de ricin, d'avocat, de jojoba, d'olive ou de germes de céréales ; des alcools tels que l'alcool oléique, l'alcool linoléique ou linolénique, l'alcool isostéarique ou l'octyl dodécanol.

On peut également citer les huiles siliconées telles que les PDMS, éventuellement phénylées telles que les phényltriméthicones.

Un tel ester peut en particulier être choisi dans le groupe constitué par l'adipate de dioctyle, le palmitate d'éthyl-2 hexyle, l'adipate de diisopropyle, l'hexanoate d'éthyl-2 hexyle, le laurate d'éthyle, le myristate de méthyle, l'octanoate d'octyldodécyle, le néopentanoate d'isodécyle, le myristate d'éthyle, le propionate de myristyle, l'éthyl-2 hexanoate d'éthyl-2 hexyle, l'octanoate d'éthyl-2 hexyle, le caprate/caprylate d'éthyl-2 hexyle, le palmitate de méthyle, le myristate de butyle, le myristate d'isobutyle, le palmitate d'éthyle, le laurate d'isohexyle, le laurate d'hexyle, l'isostéarate d'isopropyle.

Lorsque la composition est une émulsion, la phase huileuse peut être présente à raison de 5 à 95% du poids total de la composition.

La composition selon l'invention peut, en outre, comprendre :
- un agent permettant la mise en suspension de la phase grasse, par exemple un copolymère d'acrylates d'alkyle en C₁₀-C₃₀ et d'acide acrylique ou méthacrylique ou de leur ester (Pemulen® TR1, Pemulen® TR2, Carbopol® 1342 de GOODRICH) ; ou un copolymère acrylamide/acide méthylpropane sulfonique (Sepigel® de SEPPIC), et/ou
- un agent de mise en dispersion de la phase grasse, tel qu'un système émulsionnant ou vésiculaire à base de vésicules, éventuellement de taille nanométrique, constituées de lipides ioniques (liposomes) ou non ioniques, et en particulier les systèmes émulsionnants bien connus de l'homme du métier constitués de stéarate de glycéryl/PEG 100 stéarate (CTFA), d'alcool cétylique et d'alcool stéarylique, le stéarate de PEG-50, le stéarate de PEG-40, le tristéarate de sorbitane, et les stéarates de sorbitane oxyéthylénés.

La composition de l'invention peut en outre comprendre un agent pour modifier sa viscosité et obtenir des textures plus ou moins gélifiées, tel que :
- les dérivés cellulosiques (carboxyméthylcellulose, hydroxyéthylcellulose, hydroxypropylméthylcellulose),
- les gommes naturelles telles que les gommes de xanthane, de guar, de caroube, les scléroglucanes, les dérivés de chitine ou de chitosane, les carraghénanes,
- les cires ou les gommes ayant par exemple des propriétés émollientes ou lubrifiantes,
- les dérivés polycarboxyvinyliques du type Carbomer (commercialisées par la société GOODRICH sous les dénominations Carbopol® 940, 951, ou par la société 3V-SIGMA sous la dénomination Synthalen® K ou Synthalen® L).

Les compositions selon l'invention peuvent encore contenir des adjuvants couramment utilisés dans le domaine considéré, tels que des agents conservateurs, des antioxydants, des agents séquestrants, ou des agents gélifiants (notamment hydrophiles), des parfums, des charges telles que du kaolin et de l'amidon, voire les microsphères creuses, les filtres UV, les actifs de soin de la peau, en particulier les composés anti-irritants et/ou les rétinoides et/ou les (alpha) hydroxy-acides, et/ou des vitamines, et/ou des dérivés de DHEA.

Comme agents conservateurs selon l'invention, on peut citer par exemple les alkylparaben, les arylparaben, les dérivés de chlorhexidine, les alkylbenzoates, les acides salicylique, sorbique et propionique, le phénoxy éthanol, les esters d'alkyle et les sels alcalins et alcalino-terreux de ces acides.

Comme agents gélifiants hydrophiles selon l'invention, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme agents gélifiants lipophiles, on peut citer les argiles modifiées comme les bentones, les sels métalliques d'acides gras et la silice hydrophobe.

Les compositions se présentent le plus fréquemment sous forme de lait, crème, gel, ou microémulsions, d'autres modes de présentation n'étant pas exclus.

Bien entendu, l'homme du métier veillera à choisir ce ou ces éventuels composés complémentaires et/ou leur quantité, de manière telle que les propriétés avantageuses de la composition selon l'invention ne soient pas, ou substantiellement pas, altérées par l'adjonction envisagée. En particulier, ces composés ne devront pas nuire aux propriétés avantageuses de l'hydroxystilbène, ni favoriser sa cristallisation.

La composition selon l'invention peut être utilisée comme produit de soin, ou comme produit de maquillage de la peau, ou encore comme produit capillaire, tel qu'un shampooing ou un après-shampooing.

La présente invention se rapporte également à l'utilisation cosmétique de la composition selon l'invention pour prévenir ou traiter les signes du vieillissement cutané.

Enfin, la présente invention se rapporte également à un procédé de préparation d'une composition selon l'invention, caractérisée en ce qu'il comprend une étape consistant à mélanger au moins un hydroxystilbène à au moins un polyol, dans un rapport pondéral du polyol à l'hydroxystilbène d'au moins 150/1.

Les compositions selon l'invention se présentant sous la forme d'émulsions eau-dans-huile (E/H), ou huile-dans-eau (H/E), ou multiple sont préparées de manière classique par préparation des phases aqueuses et huileuses et incorporation d'une des phases dans l'autre sous agitation.

Les compositions selon l'invention se présentant sous forme d'une base oléosome sont préparées de la manière suivante :
- dans une première étape, on mélange sous agitation la phase grasse comprenant le tensioactif lipophile, le tensioactif hydrophile et l'acide gras, et la phase aqueuse comprenant l'agent basique, l'hydroxystilbène et le (ou les) polyol(s), et
- dans une deuxième étape, on soumet le mélange obtenu à une homogénéisation basée sur le principe de la cavitation.

Dans la première étape, le mélange est soumis à une agitation classique, par exemple dans un homogénéisateur tournant à une vitesse comprise entre 500 et 5000 t/mn environ, pendant un temps compris entre 10 et 60 mn environ, et à une température comprise entre 20 et 95°C environ.

Dans la deuxième étape, l'homogénéisation résulte du phénomène de cavitation créé et entretenu au sein du mélange, alors sous forme liquide, en mouvement à une vitesse linéaire d'au moins 100 m/s. Elle peut être opérée par utilisation d'un homogénéisateur haute pression fonctionnant sous des pressions comprises entre 200 et 1000 bars environ.

Le principe d'utilisation de ce type d'homogénéisateur est bien connu de l'homme de l'art. On opère par passages successifs, généralement de 2 à 10 passages, sous la pression retenue, le mélange étant ramené à pression normale entre chaque passage.

L'homogénéisation de la deuxième étape peut également être obtenue sous l'action d'ultrasons ou encore par utilisation d'homogénéisateurs équipés d'une tête de type rotor-stator.

Si l'hydroxystilbène et le (ou les) polyol(s) sont introduits à l'état libre dans la phase aqueuse, ils sont introduits au cours de la première étape.

Si, par contre, ils sont introduits dans la phase aqueuse à l'état encapsulés, ils sont introduits dans une troisième étape ultérieure, par simple mélange.

De manière préférée, l'hydroxystilbène et le (ou les) polyol(s) sont introduit(s) à l'état libre dans la phase aqueuse.

L'invention sera mieux illustrée à l'aide des exemples non limitatifs suivants.

Dans les exemples, sauf indication contraire, tous les pourcentages et parties sont exprimés en poids.

### EXEMPLES :

### Produits :

- hydroxystilbènes :
   - 3,4',5-trihydroxystilbène commercialisé par la société SIGMA sous la dénomination resvératrol®
- polyols :
   - polyéthylène glycol (8 OE)
   - butylène-1,3-glycol
   - 5-[2-(4-hydroxyphényl)vinyl]benzène-1,3-diol
- éthanol

### Emulsions :

Pour la mise en solution des polyols et du resvératrol, on utilise des émulsions huile-dans-eau (H/E), avec et sans éthanol, des émulsions eau-dans-huile (E/H), et des bases oléosomes.
- émulsions huile-dans-eau (H/E) :
   - 5 émulsions E₁, E₂, E₃, E₄ et E₅ dont les compositions sont respectivement présentées dans les tableaux 1 à 5,
- émulsions eau-dans-huile (E/H) :
   - 2 émulsions E₆ et E₇ dont les compositions sont respectivement présentées dans les tableaux 6 et 7,
- bases oléosomes :
   - 2 émulsions E₈ et E₉ dont les compositions sont respectivement présentées dans les tableaux 8 et 9.

### Emulsion E₁ (H/E)

**Tableau 1**

| Phase | Nom chimique | Quantité (%) |
|---|---|---|
| a₁ | Eau déminéralisée stérilisée | 71,8 |
| | Copolymère acide acrylique/méthacrylate de stéaryle polymérisé dans un mélange acétate d'éthyle/cyclohexane | 0,5 |
| a₂ | Butylène-1,3-glycol | 1 |
| | p-hydroxybenzoate de méthyle | 0,2 |
| b | Eau déminéralisée stérilisée | 2 |
| | Triéthanolamine à 99% | 0,3 |
| c | Néopentanoate d'isodécyle | 10 |
| | P-hydroxybenzoate de propyle | 0,1 |
| d | Polyéthylène glycol (8 OE) | 5 |
| | Butylène-1,3-glycol | 4 |
| | 5-[2-(4-hydroxyphényl)vinyl]benzène-1,3-diol | 0,1 |
| | alcool éthylique 96 degrés non dénaturé | 5 |

### Emulsion E₂ (H/E)

**Tableau 2**

| Phase | Nom chimique | Quantité (%) |
|---|---|---|
| a₁ | Eau déminéralisée stérilisée | 66,6 |
| | Sel penta-sodique de l'acide éthylène diamine tétra-méthylène phosphonique dans l'eau à 33% non stabilisé | 0,1 |
| | Copolymère acide acrylique/méthacrylate de stéaryle polymérisé dans un mélange acétate d'éthyle/cyclohexane | 0,5 |
| a₂ | Butylène-1,3-glycol | 1 |
| | p-hydroxybenzoate de méthyle | 0,2 |
| b | Eau déminéralisée stérilisée | 2 |
| | Tri-éthanolamine à 99% | 0,3 |
| c | Néopentanoate d'isodécyle | 10 |
| | P-hydroxybenzoate de propyle | 0,1 |
| d | Polyéthylène glycol (8 OE) | 7 |
| | Butylène-1,3-glycol | 7 |
| | 5-[2-(4-hydroxyphényl)vinyl]benzène-1,3-diol | 0,2 |
| | alcool éthylique 96 degrés non dénaturé | 5 |

### Emulsion E₃ (H/E)

**Tableau 3**

| Phase | Nom chimique | Quantité (%) |
|---|---|---|
| a₁ | Eau déminéralisée stérilisée | 71,6 |
| | Sel penta-sodique de l'acide éthylène diaminé tétra-méthylène phosphonique dans l'eau à 33% non stabilisé | 0,1 |
| | Copolymère acide acrylique/méthacrylate de stéaryle polymérisé dans un mélange acétate d'éthyle/cyclohexane | 0,5 |
| a₂ | Butylène-1,3-glycol | 1 |
| | p-hydroxybenzoate de méthyle | 0,2 |
| b | Eau déminéralisée stérilisée | 2 |
| | Triéthanolamine à 99% | 0,3 |
| c | Néopentanoate d'isodécyle | 10 |
| | P-hydroxybenzoate de propyle | 0,1 |
| d | Polyéthylène glycol (8 OE) | 7 |
| | Butylène-1,3-glycol | 7 |
| | 5-[2-(4-hydroxyphényl)vinyl]benzène-1,3-diol | 0,2 |

### Emulsion E₄ (H/E)

**Tableau 4**

| Phase | Nom chimique | Quantité (%) |
|---|---|---|
| a | Tristéarate de sorbitane | 0,9 |
| | Stéarate de polyéthylène glycol (40 OE) | 2 |
| | Alcool cétylique pur, d'origine naturelle | 4 |
| | Mono,di,tri-palmito-stéarate de glycéryle | 3 |
| | Myristate de myristyle | 2 |
| | Palmitate d'éthyl-2-hexyle | 2 |
| | Isoparaffine (6-8 moles d'isobutylène) hydrogéné (viscosité : 34 cst à 25°C) | 3 |
| | Alcool héxyl-2 décylique-1 | 5 |
| | p-hydroxybenzoate de propyle | 0,15 |
| b | Eau déminéralisée stérilisée | 43,7 |
| | P-hydroxybenzoate de méthyle | 0,25 |
| c | Cyclopentadiméthylsiloxane (viscosité : 4 cst) | 10 |
| d | Polyéthylène glycol (8 OE) | 9 |
| | Butylène-1,3-glycol | 9 |
| | 5-[2-(4-hydroxyphényl)vinyl]benzène-1,3-diol | 0,2 |
| e | Eau déminéralisée stérilisée | 5 |
| | Imidazolidinyl urée | 0,3 |
| | Acide polyacrylamidométhyl propane sulfonique neutralisé partiellement à l'ammoniaque et hautement réticulé | 0,5 |

### Emulsion E₅ (H/E)

**Tableau 5**

| Phase | Nom chimique | Quantité (%) |
|---|---|---|
| a | Tristéarate de sorbitane | 0,9 |
| | Stéarate de polyéthylène glycol (40 OE) | 2 |
| | Alcool cétylique pur, d'origine naturelle | 4 |
| | Mono,di,tri-palmito-stéarate de glycéryle | 3 |
| | Myristate de myristyle | 2 |
| | Palmitate d'éthyl-2-héxyle | 2 |
| | Iso-paraffine (6-8 moles d'isobutylène) hydrogéné (viscosité : 34 cst à 25°C) | 3 |
| | Alcool héxyl-2-décylique-1 | 5 |
| | p-hydroxybenzoate de propyle | 0,15 |
| b | Eau déminéralisée stérilisée | 45,7 |
| | P-hydroxybenzoate de méthyle | 0,25 |
| c | Cyclopentadiméthylsiloxane (viscosité : 4 cst) | 10 |
| d | Polyéthylène glycol (8 OE) | 8 |
| | Butylène-1,3-glycol | 8 |
| | 5-[2-(4-hydroxyphényl)vinyl]benzène-1,3-diol | 0,2 |
| e | Eau déminéralisée stérilisée | 5 |
| | Imidazolidinyl urée | 0,3 |
| | Acide polyacrylamidométhyl propane sulfonique neutralisé partiellement à l'ammoniaque et hautement réticulé | 0,5 |

### Emulsion E₆ (E/H)

**Tableau 6**

| Phase | Nom chimique | Quantité (%) |
|---|---|---|
| a | Polyméthylcétyldiméthylméthylsiloxane oxyéthyléné (20/75-5-viscosité 3000 cst) | 1,5 |
| | Isostéarate polyglycérole (4 moles) | 0,5 |
| | Isohexadécane | 5 |
| | Octyl-2-dodécanol | 8 |
| | Mélange de stéarate d'éthylène glycol acétyle, tristéarate de glycéryle | 1 |
| | P-hydroxybenzoate de propyle | 0,15 |
| | Huile d'amandes d'abricot désodorisée (triglycérides d'acides oléique-linoléique 66/28) raffinée non stabilisée | 5 |
| b | Eau déminéralisée stérilisée | 67,4 |
| | P-hydroxybenzoate de méthyle | 0,25 |
| | Sulfate de magnésium,7 H₂O | 0,7 |
| | Sel disodique de l'acide éthylène diamine tétraacétique,2 H₂O | 0,1 |
| c | Eau déminéralisée stérilisée | 5 |
| | Imidazolidinyl urée | 0,3 |
| d | Polyéthylène glycol (8 OE) | 5 |
| | 5-[2-(4-hydroxyphényl)vinyl]benzène-1,3-diol | 0,1 |

### Emulsion E₇ (E/H)

**Tableau 7**

| Phase | Nom chimique | Quantité (%) |
|---|---|---|
| a | Polyméthylcétyldiméthylméthylsiloxane oxyéthyléné (20/75-5-viscosité 3000 cst) | 1,5 |
| | Isostéarate polyglycérole (4 moles) | 0,5 |
| | Isohexadécane | 5 |
| | Octyl-2-dodécanol | 8 |
| | Mélange de stéarate d'éthylène glycol acétyle, tristéarate de glycéryle | 1 |
| | P-hydroxybenzoate de propyle | 0,15 |
| | Huile d'amandes d'abricot désodorisée (triglycérides d'acides oléique-linoléique 66/28) raffinée non stabilisée | 5 |
| b | Eau déminéralisée stérilisée | 56,3 |
| | P-hydroxybenzoate de méthyle | 0,25 |
| | Sulfate de magnésium,7 H₂O | 0,7 |
| | Sel disodique de l'acide éthylène diamine tétraacétique,2 H₂O | 0,1 |
| c | Eau déminéralisée stérilisée | 5 |
| | Imidazolidinyl urée | 0,3 |
| d | Polyéthylène glycol (8 OE) | 8 |
| | Butylène-1,3-glycol | 8 |
| | 5-[2-(4-hydroxyphényl)vinyl]benzène-1,3-diol | 0,2 |

### Emulsion E₈ (base oléosome)

**Tableau 8**

| Phase | Nom chimique | Quantité (%) |
|---|---|---|
| a | Distéarate polyglycérole (2 moles) | 2 |
| | Monostéarate de polyéthylène glycol (8 OE) | 1,35 |
| | Acide stéarique (triple pression) (C₁₆-C₁₈ : 50/50) | 1 |
| | Stéarate d'isocétyle | 7 |
| | Perhydrosqualène végétal raffiné | 13 |
| | Di-tertio-butyl hydroxy-4-toluène | 0,07 |
| b₁ | Polyéthylène glycol (8 OE) | 5 |
| | 5-[2-(4-hydroxyphényl)vinyl]benzène-1,3-diol | 0,1 |
| b₂ | Eau déminéralisée stérilisée | 48,68 |
| | Tri-éthanolamine à 99% | 0,25 |
| | Phénoxy-2 éthanol | 1 |
| | Chlorphénésine | 0,25 |
| | Alcool phényléthylique | 0,25 |
| | Sel pentasodique de l'acide éthylène diamine tétraméthylène phosphonique dans l'eau à 33% non stabilisée | 0,05 |
| c | Eau déminéralisée stérilisée | 19 |
| | Acide polyacrylamidométhyl propane sulfonique neutralisé partiellement à l'ammoniaque et hautement réticulé | 1 |

### Emulsion E₉ (base oléosome)

**Tableau 9**

| Phase | Nom chimique | Quantité (%) |
|---|---|---|
| a | Distéarate polyglycérole (2 moles) | 2 |
| | Monostéarate de polyéthylène glycol (8 OE) | 1,35 |
| | Acide stéarique (triple pression) (C₁₆-C₁₈ :50/50) | 1 |
| | Stéarate d'iso-cétyle | 7 |
| | Perhydrosqualène végétal raffiné | 13 |
| | Di-tertio-butyl hydroxy-4 toluène | 0,07 |
| b | Eau déminéralisée stérilisée | 37,58 |
| | Triéthanolamine à 99% | 0,25 |
| | Phénoxy-2-éthanol | 1 |
| | Chlorphénésine | 0,25 |
| | Alcool phényléthylique | 0,25 |
| | Sel pentasodique de l'acide éthylène diamine tétraméthylène phosphonique dans l'eau à 33% non stabilisée | 0,05 |
| c | Eau déminéralisée stérilisée | 19 |
| | Acide polyacrylamidométhyl propane sulfonique neutralisé partiellement à l'ammoniaque et hautement réticulé | 1 |
| d | Polyéthylène glycol (8 OE) | 8 |
| | Butylène-1,3-glycol | 8 |
| | 5-[2-(4-hydroxyphényl)vinyl]benzène-1,3-diol | 0,2 |

### Exemple 1 : Solubilisation en émulsion H/E

On a ajouté aux émulsions E₁ à E₅ du resvératrol sous la forme et dans les quantités indiquées au tableau 10.

La stabilité physico-chimique des émulsions obtenues est vérifiée par un contrôle macroscopique et microcospique, au bout de 24 heures et au-delà.

Le comportement du resvératrol lors de la solubilisation dans les émulsions E₁ à E₅, ainsi que l'évolution dans le temps, sont reportés dans le tableau 10 ci-après.

**Tableau 10**

| Emulsions H/E | Stabilité physico-chimique | | |
|---|---|---|---|
| | 24 heures | 1 mois | 2 mois |
| E₁ + 0,1% de resvératrol pur [polyols]/[resvératrol]=100/1 | cristaux visibles au microscope | - | - |
| E₂ + 0,2% de resvératrol à 52,5% de matière active [polyols]/[resvératrol]=150/1 | - | - | pas de cristaux à 4°C ni à 25°C |
| E₃ + 0,2% de resvératrol à 52,5% de matière active [polyols]/[resvératrol]=150/1 | - | cristaux à 4°C | - |
| E₄ + 0,2% de resvératrol à 52,5% de matière active [polyols]/[resvératrol]=180/1 | - | - | pas de cristaux à 25°C |
| E₅ + 0,2% de resvératrol à 52,5% de matière active [polyols]/[resvératrol]=160/1 | - | - | pas de cristaux à 25°C |

Le tableau 10 montre que les polyols permettent une bonne solubilisation du resvératrol dans les émulsions H/E, lorsque le rapport pondéral des polyols au resvératrol est d'au moins 150/1.

La présence d'éthanol, associé aux polyols dans la composition, améliore encore la solubilisation.

### Exemple 2 : Solubilisation en émulsion E/H

On a ajouté aux émulsions E₆ et E₇ du resvératrol sous la forme et dans les quantités indiquées au tableau 11.

La stabilité physico-chimique des émulsions obtenues est, comme à l'exemple 1, vérifiée par un contrôle macroscopique et microcospique, au bout de 24 heures et au-delà.

Le comportement du resvératrol lors de la solubilisation dans les émulsions E₆ et E₇, ainsi que l'évolution dans le temps, sont reportés dans le tableau 11 ci-après.

**Tableau 11**

| Emulsions E/H | Stabilité physico-chimique | |
|---|---|---|
| | 24 heures | 2 mois |
| E₆ + 0,1% de resvératrol pur [polyols]/[resvératrol]=50/1 | cristaux à température ambiante | - |
| E₇ + 0,2% de resvératrol à 52,5% de matière active [polyols]/[resvératrol]=160/1 | - | pas de cristaux à 25°C |

Le tableau 11 montre que les polyols permettent une bonne solubilisation du resvératrol dans des émulsions E/H lorsque le rapport pondéral est polyol au resvératrol est d'au moins 150/1.

### Exemple 3 : Solubilisation dans une base oléosome

On a ajouté aux émulsions E₈ et E₉ du resvératrol sous la forme et dans les quantités indiquées au tableau 12.

La stabilité physico-chimique des émulsions obtenues est, comme aux exemples 1 et 2, vérifiée par un contrôle macroscopique et microcospique, au bout de 24 heures et au-delà.

Le comportement du resvératrol lors de la solubilisation dans les émulsions E₈ et E₉, ainsi que l'évolution dans le temps, sont reportés dans le tableau 12 ci-après :

**Tableau 12**

| Base oléosome | Stabilité physico-chimique | |
|---|---|---|
| | 1 mois | 2 mois |
| E₈ + 0,1% de resvératrol pur [polyols]/[resvératrol]=50/1 | cristaux à 25°C | - |
| E₉ + 0,2% de resvératrol à 52,5% de matière active [polyols]/[resvératrol]=160/1 | - | pas de cristaux à 25°C |

Le tableau 12 montre que les polyols permettent une bonne solubilisation du resvératrol dans des bases oléosomes lorsque le rapport pondéral des polyols au resvératrol est d'au moins 150/1.

## Revendications

1. Composition adaptée à une application topique sur la peau comprenant, dans un milieu physiologiquement acceptable, au moins un hydroxystilbène et au moins un polyol, dans un rapport pondéral du polyol à l'hydroxystilbène d'au moins 150/1.

2. Composition selon la revendication 1, **caractérisée en ce que** l'hydroxystilbène est choisi parmi :
- le 4'-hydroxystilbène,
- le 2',4'-dihydroxystilbène,
- le 3',4'-dihydroxystilbène,
- le 4,4'-dihydroxystilbène,
- le 2',4',4-trihydroxystilbène,
- le 3',4',4-trihydroxystilbène,
- le 2,4,4'-trihydroxystilbène,
- le 3,4,4'-trihydroxystilbène,
- le 3,4',5-trihydroxystilbène,
- le 2',3,4-trihydroxystilbène,
- le 2,3',4-trihydroxystilbène,
- le 2',2,4'-trihydroxystilbène,
- le 2,4,4',5-tétrahydroxystilbène,
- le 2',3,4',5-tétrahydroxystilbène,
- le 2,2',4,4'-tétrahydroxystilbène,
- le 3,3',4',5-tétrahydroxystilbène,
- le 2,3',4,4'-tétrahydroxystilbène,
- le 3,3',4,4'-tétrahydroxystilbène,
- le 3,3',4',5,5'-pentahydroxystilbène,
- le 2,2',4,4',6-pentahydroxystilbène,
- le 2,3',4,4',6-pentahydroxystilbène, et
- le 2,2',4,4',6,6'-hexahydroxystilbène.

3. Composition selon la revendication 1 ou 2, **caractérisée en ce que** l'hydroxystilbène est le 3,4',5 trihydroxystilbène.

4. Composition selon l'une quelconque des revendications précédentes **caractérisée en ce que** l'hydroxystilbène est présent en une quantité allant de 0,001% à 10% en poids, et de préférence de 0,005% à 0,5% en poids par rapport au poids total de la composition.

5. Composition selon l'une quelconque des revendications précédentes, **caractérisée en ce que** les polyols sont choisis parmi la glycérine, les glycols, les polyéthylènes glycols et leurs mélanges.

6. Composition selon la revendication 5, **caractérisée en ce que** les polyols sont choisis parmi le polyéthylène glycol, le butylène-1,3-glycol et le 5-[2-(4-hydroxyphényl)vinyl]benzène-1,3-diol.

7. Composition selon l'une quelconque des revendications précédentes, **caractérisée en ce qu'**elle comprend en outre un alcanol en C₁-C₆, de préférence l'éthanol.

8. Composition selon la revendication 7, **caractérisée en ce que** l'alcanol représente jusqu'à 10% en poids, de préférence jusqu'à 5% en poids du poids total de la composition.

9. Composition selon l'une quelconque des revendications précédentes, **caractérisée en ce qu'**elle est constituée par une émulsion du type huile-dans-eau (H/E) ou eau-dans-huile (E/H), une nanoémulsion, une microémulsion, un gel aqueux, un gel anhydre, une solution, ou par une émulsion multiple.

10. Composition selon l'une quelconque des revendications 1 à 8, **caractérisée en ce qu'**elle est constituée par une émulsion du type huile-dans-eau formée de globules huileux pourvus d'un enrobage cristal liquide lamellaire dispersés dans une phase aqueuse.

11. Composition selon la revendication 10, **caractérisée en ce que** les globules huileux ont un diamètre moyen inférieur à 500 nanomètres, et de préférence inférieur à 300 nanomètres.

12. Composition selon la revendication 10 ou 11, **caractérisée en ce que** l'enrobage cristal liquide lamellaire est une couche monolamellaire ou oligolamellaire obtenue à partir d'au moins un agent tensioactif lipophile, d'au moins un agent tensioactif hydrophile et d'au moins un acide gras.

13. Composition selon l'une quelconque des revendications 10 à 12, **caractérisée en ce que** la phase aqueuse contient à l'état dissous l'hydroxystilbène et le polyol.

14. Composition selon l'une quelconque des revendications précédentes, **caractérisée en ce qu'**elle comprend une phase huileuse à base d'huile animale, végétale, minérale, siliconée, fluorée et/ou synthétique.

15. Composition selon la revendication 14, **caractérisée en ce que** la phase huileuse comprend également au moins un alcool gras ou au moins un acide gras, et au moins un tensioactif.

16. Composition selon l'une quelconque des revendications précédentes, **caractérisée en ce qu'**elle comprend au moins un adjuvant choisi parmi les conservateurs, les parfums, les charges, les filtres UV, les actifs de soin de la peau, en particulier les composés anti-irritants et/ou les rétinoides et/ou les (alpha) hydroxy-acides.

17. Procédé de préparation d'une composition telle que définie selon l'une quelconque des revendications 1 à 16, **caractérisé en ce qu'**il comprend une étape consistant à mélanger au moins un hydroxystilbène à au moins un polyol, dans un rapport pondéral du polyol à l'hydroxystilbène d'au moins 150/1.

18. Procédé selon la revendication 17, **caractérisé en ce que**, dans le cas d'une composition constituée d'une émulsion de type huile-dans-eau telle que définie selon la revendication 10, il comprend les deux étapes suivantes:
- une première étape de mélange sous agitation de la phase huileuse comprenant le tensioactif lipophile, le tensioactif hydrophile et l'acide gras, avec la phase aqueuse comprenant l'agent basique, le polyol et l'hydroxystilbène, et
- une deuxième étape d'homogénéisation du mélange obtenu au cours de la première étape.

19. Procédé selon la revendication 18, **caractérisé en ce que** l'homogénéisation est obtenue soit à l'aide de hautes pressions comprises entre 200 et 1000 bars, soit à l'aide d'ultrasons, soit à l'aide d'homogénéisateurs équipés d'une tête de rotor-stator.

20. Utilisation de la composition telle que définie selon les revendications 1 à 16 pour la fabrication de produits de soin et/ou de maquillage et/ou capillaires.

21. Utilisation cosmétique de la composition telle que définie selon les revendications 1 à 16 pour prévenir ou traiter les signes du vieillissement cutané.
